# EUROPÄISCHE PATENTSCHRIFT

(11) **EP 1 940 326 B1**
(45) Veröffentlichungstag und Bekanntmachung des Hinweises auf die Patenterteilung: **21.07.2010**
(21) Anmeldenummer: 06805408.9
(22) Anmeldetag: 12.10.2006
(51) Int. Cl.: A61F 2/76, G01L 1/22, G01L 5/16

(54) **SENSORANORDNUNG FÜR DIE MESSUNG VON KRÄFTEN UND/ODER MOMENTEN UND VERWENDUNG DER SENSORANORDNUNG**
SENSOR ASSEMBLY FOR MEASURING FORCES AND/OR TORQUES AND USE OF SAID ASSEMBLY
DISPOSITIF DE DETECTION DESTINE A LA MESURE DE FORCES ET/OU DE MOMENTS ET UTILISATION

(30) Priorität: 26.10.2005 DE 102005051495
(43) Veröffentlichungstag der Anmeldung: 09.07.2008
(73) Patentinhaber: Otto Bock Healthcare GmbH, 37115 Duderstadt (DE)
(72) Erfinder: PUSCH, Martin, 37115 Duderstadt (DE)
(74) Vertreter: Lins, Edgar
(86) Internationale Anmeldenummer: PCT/DE2006/001791
(87) Internationale Veröffentlichungsnummer: WO 2007/048375

(56) Entgegenhaltungen:
- EP-A1- 1 559 384
- DE-A1- 3 701 372
- DE-A1- 10 013 059
- DE-A1- 10 139 333
- US-A- 4 640 138

## Beschreibung

Die Erfindung betrifft eine Sensoranordnung für die Messung von Kräften und/oder Momenten, die als starrer Übertrager zur Übertragung der Kräfte und/oder Momente ausgebildet ist, einen ersten Teil mit einem ersten Anschluss und einen zweiten Teil mit einem zweiten Anschluss aufweist und mit mechanisch-elektrischen Sensorelementen zur Umsetzung mechanischer Parameter in elektrische Parameter ausgestattet ist.

Die Erfindung betrifft ferner eine Verwendung einer derartigen Sensoranordnung in einem künstlichen Glied, insbesondere in einem künstlichen Bein.

Es sind Sensoranordnungen bekannt, mit denen eine Ganganalyse für einen Patienten durchgeführt werden soll. Da die Sensoranordnungen sehr großvolumig sind, müssen sie in Testprothesen eingebaut werden, die den in der Praxis benutzten Prothesen möglichst gut entsprechen sollen. Aufgrund der anderen Konstruktion der Testprothese und der durch die Sensoranordnung veränderten Gewichtsverhältnisse sind jedoch nur Ganganalysen möglich, die dem Gangverhalten mit einer in der Praxis benutzten Prothese u. U. nur sehr unvollkommen entsprechen.

Es ist ferner versucht worden, den Patienten an der mit einer Prothese versorgten Extremität mit einer Sensoranordnung zu versehen, die mit Beschleunigungsmessern aufgebaut ist. Eine derartige Anordnung kann für die Analyse der Gangdynamik verwendbar sein, erlaubt jedoch keine Messung von Belastungen der Prothese im Stand oder bei quasi statischen Bewegungen.

Durch die DE 101 39 333 A1 ist eine Prothese mit einem Oberschenkelteil, einem Kniegelenk, einem Unterschenkelteil und einem Fußteil bekannt, bei der das Unterschenkelteil zur Aufnahme einer Sensoranordnung ausgebildet ist. Die Sensoranordnung ist zur messtechnischen Trennung von axialen Belastungen von Biegebelastungen eingerichtet. Sie besteht aus einem Rahmen mit gebogenen Seitenstegen zur Messung der Biegebelastung und einem mittigen dünnen Steg zur Messung der vertikalen (axialen) Belastung.

Ein Bedürfnis, Kräfte und/oder Drehmomente zu bestimmen, besteht nicht nur für Prothesen, sondern für zahlreiche andere Anwendungsgebiete, beispielsweise für die Auslegung und Steuerung von Roboterarmen.

Der vorliegenden Erfindung liegt daher die Aufgabe zugrunde, eine Sensoranordnung der eingangs erwähnten Art zu schaffen, die auf kleinem Raum aufbaubar ist und eine zuverlässige Messung von statischen wie dynamischen Belastungen ermöglicht.

Diese Aufgabe wird erfindungsgemäß mit einer Sensoranordnung der eingangs erwähnten Art gelöst, die dadurch gekennzeichnet ist, dass die Sensoranordnung ausgehend vom ersten Anschluss einen den ersten Anschluss umgebenden ersten Flansch aufweist, der über senkrecht zum ersten Flansch stehende Stege mit einem parallel zum ersten Flansch ausgerichteten zweiten Flansch verbunden ist, wobei der Zwischenraum zwischen den Stegen größer als Breitenabmessungen der Stege ist, dass auf dem zweiten Flansch der zweite Anschluss angeordnet ist und dass die Sensorelemente zur Bestimmung von Dehnungen oder Stauchungen ausgelegt und auf wenigstens einer der beiden Oberflächen des zweiten Flansches neben den Stegen angeordnet sind.

Die erfindungsgemäße Sensoranordnung erlaubt eine sichere Übertragung der Kräfte von dem ersten Anschluss zum vorzugsweise zentrisch auf dem zweiten Flansch angeordneten zweiten Anschluss und umgekehrt, wobei die übertragenen Kräfte durch Messung von kleinsten Verformungen an dem zweiten Flansch erkennbar sind. Aufgrund der Übertragung der Kräfte zwischen den beiden Flanschen mittels der Stege werden die Verformungen in den Bereichen der den Stegen benachbarten Oberfläche des zweiten Flansches konzentriert und können dort mit den auf Dehnung oder Stauchung reagierenden Sensorelementen detektiert werden. Die Stege, deren Länge vorzugsweise ihre Breitenabmessungen übersteigt, bewirken eine Entkopplung der beiden Flansche, die einer Übertragung der Kräfte ermöglicht, andererseits die Verformungen auf einen der Flansche, hier auf den zweiten Flansch, konzentriert, sodass bezüglich der Verformungen, also bezüglich des Messeffektes, eine Entkopplung der beiden Flansche erreicht wird.

In einer bevorzugten Ausführungsform der Erfindung sind die Sensorelemente zur Aufnahme von Dehnungen und Stauchungen auf beiden Oberflächen des zweiten Flansches angeordnet. Durch die dadurch ermöglichte Differenzierung zwischen Dehnungen und Stauchungen kann die Art der übertragenen Kräfte und/oder Momente mit höherer Zuverlässigkeit bestimmt werden. Ferner ist es möglich, vergrößerte Messsignale zu erhalten, wenn die auf der Oberseite und auf der Unterseite des zweiten Flansches befindlichen Sensorelemente in geeigneter Weise miteinander verschaltet werden.

In einer bevorzugten Ausführungsform der Erfindung ist die Sensoranordnung zweiteilig mit einem den ersten Flansch aufweisenden ersten Teil und einen den zweiten Flansch und die Stege aufweisenden zweiten Teil aufgebaut, wobei die beiden Teile starr miteinander verbindbar sind, beispielsweise durch eine Schraubverbindung. Die Schraubverbindung kann bevorzugt über die Stege erfolgen, wenn diese mit einer Gewindebohrung zur Aufnahme einer Befestigungsschraube versehen sind.

Der erste Anschluss der Sensoranordnung ist vorzugsweise hutförmig mit einem zylindrischen Querschnitt ausgebildet, an den sich der erste Flansch anschließt. Der hutförmige Anschluss kann vorzugsweise eine bekannte Justierpyramide übergreifen und mit Durchgangs-Gewindebohrungen für an den Planflächen der Justierpyramide angreifenden Justierschrauben versehen sein.

Der zweite Anschluss kann beispielsweise mit einer Justierpyramide ausgebildet sein, die über eine konvexe Wölbung in den zweiten Flansch übergeht.

Vorzugsweise sind die Sensorelemente Dehnungsmessstreifen, die in einer bevorzugten Ausführungsform der Erfindung zur Aufnahme linienförmiger Dehnungen oder Stauchungen positioniert sind.

Die vorzugsweise linienförmigen Dehnungs- bzw. Stauchungsbereiche können auf dem zweiten Flansch durch die Anbringung von Ausnehmungen in ihrer Position beeinflusst werden. Die Ausnehmungen können kreisrunde Bohrungen, aber auch Durchgangsöffnungen mit einer beliebigen, beispielsweise etwa dreieckigen Außenkontur sein.

Vorzugsweise weisen der erste und der zweite Flansch eine viereckige Kontur auf und es sind vier Stege zur Verbindung der beiden Flansche an deren Ecken vorgesehen. Die Ausnehmungen, die zur Positionierung der Dehnungs- bzw. Stauchungsbereiche in dem zweiten Flansch vorgesehen sind, befinden sich vorzugsweise zwischen dem zweiten Anschluss und den Stegen, also in einer diagonalen Richtung des vorzugsweise quadratischen zweiten Flansches.

Mit einer derartigen Konstruktion gelingt es, parallel zu den Kanten des zweiten Flansches von den Ausnehmungen verlaufende linienförmige Dehnungs- oder Stauchungsbereiche parallel zu den Kanten des zweiten Flansches auszubilden, sodass hierfür die Dehnungsmessstreifen positioniert werden.

Die erfindungsgemäße Sensoranordnung lässt sich auf einem sehr kleinen Raum realisieren und benötigt eine Bauhöhe von nicht mehr als 2 bis 3 cm.

Die erfindungsgemäße Sensoranordnung lässt sich somit an die Größe und das Gewicht beispielsweise eines Rotationsadapters anpassen, der in einer Beinprothese unmittelbar oberhalb des künstlichen Kniegelenks angeordnet wird. Der eine zusätzliche Rotation ermöglichende Rotationsadapter einer derartigen Prothese kann gegen die erfindungsgemäße Sensoranordnung ohne weiteres ausgetauscht werden, sodass die für eine Ganganalyse benötigten Kräfte und Momente an der für den Patienten für den anschließenden Gebrauch hergestellten Prothese bestimmt werden können. Die Bestimmung der Belastungen kann somit an der endgültigen Prothese vorgenommen werden, wobei eine Optimierung durch Justierungen der Prothese möglich ist.

Die erfindungsgemäße Sensoranordnung ist auch für einen Langzeit-Einbau in eine Prothese geeignet, um Langzeituntersuchungen durchzuführen.

Die Erfindung soll im Folgenden anhand eines in der Zeichnung dargestellten Ausführungsbeispiels näher erläutert werden. Es zeigen:
- Figur 1: eine perspektivische Seitenansicht auf ein erstes Teil einer Sensoranordnung;
- Figur 2: eine perspektivische Ansicht von schräg unten auf das erste Teil gemäß Figur 1;
- Figur 3: eine perspektivische Seitenansicht des zweiten Teils einer Sensoranordnung;
- Figur 4: eine perspektivische Ansicht von schräg unten des zweiten Teils gemäß Figur 3;
- Figur 5: eine perspektivische Seitenansicht der aus den beiden Tei- len zusammengesetzten Sensoranordnung;
- Figur 6: eine perspektivische Ansicht von schräg unten der Sensoranordnung gemäß Figur 5;
- Figur 7: eine schematische Schnittdarstellung der Sensoranordnung für eine axiale Belastung (z-Richtung) mit einer schemati- schen Darstellung linienförmiger Stauchbereiche auf dem zweiten Flansch;
- Figur 8: eine Darstellung gemäß Figur 7 für ein Drehmoment um eine horizontale Achse (x-Achse);
- Figur 9: eine schematische Darstellung gemäß Figur 7 für eine ein- wirkende Seitenkraft;
- Figur 10: eine schematische Darstellung gemäß Figur 7 für ein Dreh- moment um eine vertikale Achse (z-Achse).

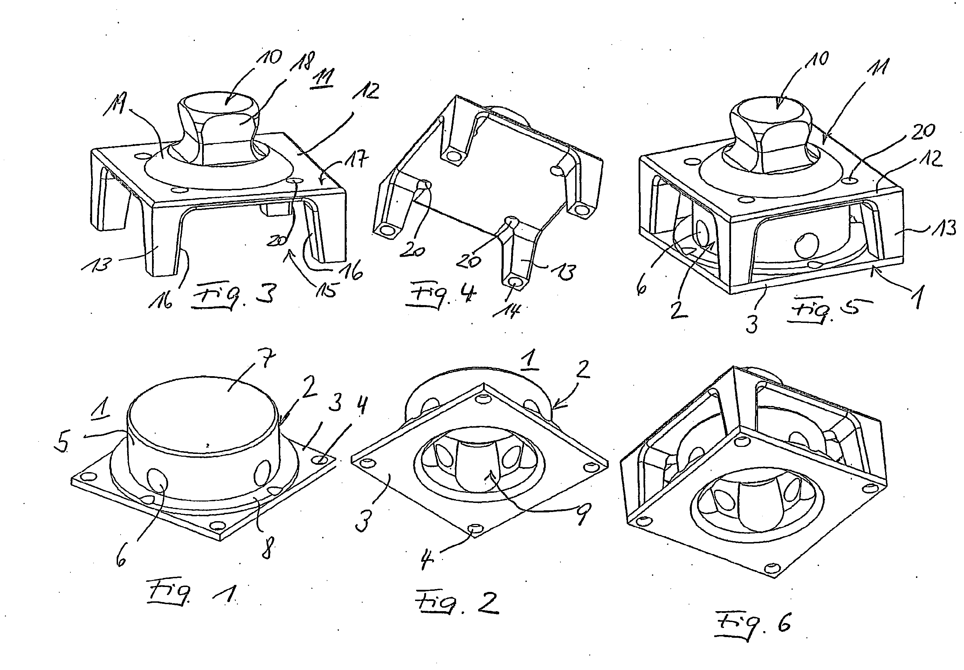

Die Figuren 1 bis 6 verdeutlichen den Aufbau eines Ausführungsbeispiels einer erfindungsgemäßen Sensoranordnung. Diese besteht aus einem ersten Teil 1, das aus einem hutförmigen zylindrischen Anschluss 2 und einem sich daran anschließenden quadratischen Flansch 3 aufgebaut ist. Der quadratische Flansch 3 weist an seinen Ecken Durchgangslöcher 4 für (nicht dargestellte) Befestigungsschrauben auf.

Der hutförmige Anschluss 2 ist mit einer zylindrischen Mantelwandung 5 aufgebaut, in der sich, jeweils um einen Drehwinkel von 90° versetzt, Gewindebohrungen 6 befinden. Der hutförmige Anschluss 2 weist an seiner Oberseite einen kreiszylindrischen Boden 7 und an seiner Unterseite eine kreisringförmige Krempe 8 auf, die einstückig mit dem rechteckigen Flansch 3 verbunden ist und diesen verstärkt.

Figur 2 verdeutlicht, dass der hutförmige Anschluss 2 einen angenähert rechteckig ausgebildeten Aufnahmeraum 9 aufweist, der zur Aufnahme eines Justieradapters dient der vier schräg liegende Justierflächen aufweist, gegen die die durch die Gewindebohrungen 6 hindurch geschraubten Justierschrauben drücken.

Ein derartiger Justieradapter 10 ist an einem zweiten Teil 11 der Sensoranordnung ausgebildet. Das zweite Teil 11 weist einen zweiten, quadratischen Flansch 12 auf, dessen Abmessungen den Abmessungen des ersten Flansches 3 entsprechen. Die beiden Flansche 3, 12 werden durch am zweiten Teil einstückig angeformte Stege 13 miteinander verbunden, die sich an den Ecken des zweiten Flansches 12 nach unten erstrecken, sodass die Stege radial außen von dem hutförmigen Anschluss 12 auf dem ersten Flansch 3 aufliegen. Die Stege 13 sind jeweils mit einer Gewinde-Sackbohrung von ihrer Unterseite aus versehen, die mit den Durchgangsbohrungen 4 des ersten Flansches 3 fluchten können.

Die Figuren 3 und 4 lassen noch erkennen, dass die Stege 13 einen rechteckigen Querschnitt aufweisen und sich zu ihren freien Enden hin, also nach unten durch eine zu einem Zwischenraum 15 zwischen zwei Stegen 13 zeigende Schräge 16 verjüngen.

Der Justieradapter 10 befindet sich auf der von den Stegen 13 abgewandten Oberseite 17 des zweiten Flansches 12. Er ist in an sich bekannter Weise in Form eines umgekehrten Pyramidenstumpfes ausgebildet und weist somit vier schräg verlaufende plane Justierflächen 18 auf, die zum Zwecke der Justierung mit Justierschrauben zusammenwirken können. Der Justieradapter 10 geht in einen Sockel 19 mit vergrößertem Durchmesser über, der mit einer gewölbten Fläche einen Übergang zu dem quadratischen zweiten Flansch 12 herstellt.

Der Justieradapter 10 bildet einen zweiten Anschluss der Sensoranordnung. Zwischen diesem zweiten Anschluss 10 und den in den Ecken des zweiten Flansches 12 angeordneten Stegen 13 befindet sich, somit in Diagonalrichtung des zweiten quadratischen Flansches 12, jeweils eine Ausnehmung 20 in Form einer Durchgangsbohrung, durch die die unten näher beschriebene Ausbildung von Spannungs- bzw. Dehnungsbereichen beeinflussbar ist.

Die Figuren 5 und 6 zeigen die aus den beiden Teilen 1, 11 zusammengesetzte Sensoranordnung im montierten Zustand (jedoch ohne Befestigungsschrauben). Es ist erkennbar, dass zwischen dem Aufnahmeraum 9 des ersten Anschlusses 2 und dem den zweiten Anschluss bildenden Justieradapter 10 nur eine geringe Bauhöhe von 2 bis 3 cm benötigt werden.

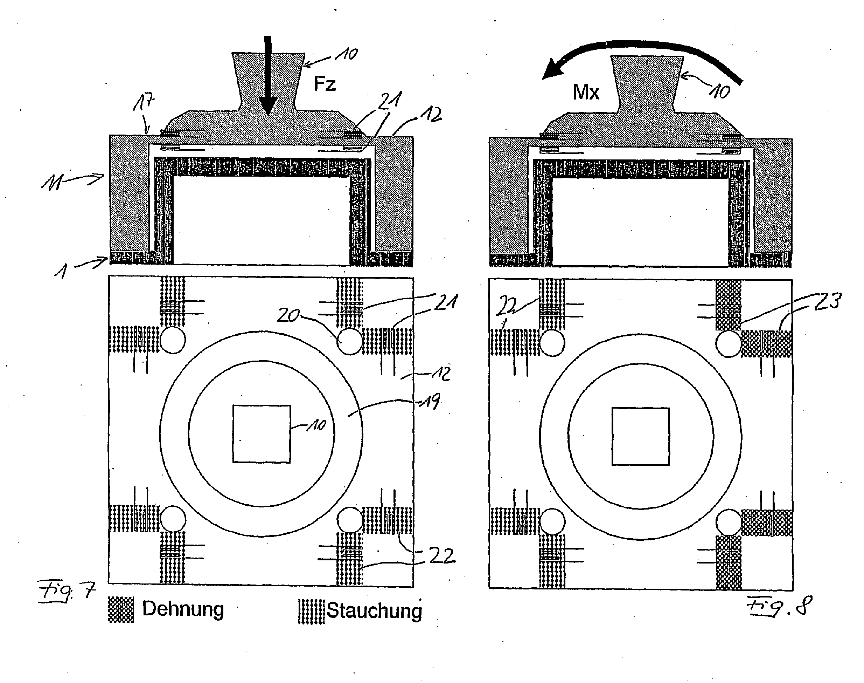

Die Figuren 7 bis 10 zeigen schematisch jeweils einen Vertikalschnitt durch die Sensoranordnung gemäß den Figuren 1 bis 6, jedoch mit einer schematischen Darstellung der auf beide Oberflächen des Flansches 12 geklebten Dehnungsmessstreifen 21 als Sensorelemente.

Die in den Figuren 7 bis 10 jeweils darunter befindliche Draufsicht verdeutlicht die Positionierung der Dehnungsmessstreifen 21 derart, dass sie durch linienförmige Stauchungsbereiche 22 oder Dehnungsbereiche 23 in ihrer Länge verändert werden, sodass sich ein geänderter Widerstand ergibt.

Figur 7 verdeutlicht den Fall einer Krafteinwirkung in z-Richtung, also in Axialrichtung einer Rohrskelettprothese für einen Unterschenkel. Die an der Oberseite 17 des zweiten Flansches 12 befindlichen Dehnungsmessstreifen 21 befinden sich dabei in Stauchungsbereichen 22, die sich linienförmig von den Durchgangsbohrungen 20 parallel zu den Kanten des zweiten Flansches 12 jeweils zur benachbarten Kante hin erstrecken. Die dementsprechend ausgerichteten Dehnungsmessstreifen 21 verändern somit ihren Widerstandswert in Richtung Stauchung.

Gemäß Figur 8 wird der Justieradapter 10 mit einem Drehmoment um eine senkrecht zur Zeichenebene (x-Richtung) stehende Achse beaufschlagt. Das Drehmoment führt für die auf der Oberseite 17 befindlichen Dehnungsmessstreifen 21 auf der Seite, zu der das Drehmoment hin gerichtet ist (vgl. den eingezeichneten Pfeil Mx in Figur 8) zu einer Stauchung, während sie auf der gegenüberliegenden Seite zur Ausbildung von Dehnungsbereichen 23 führt.

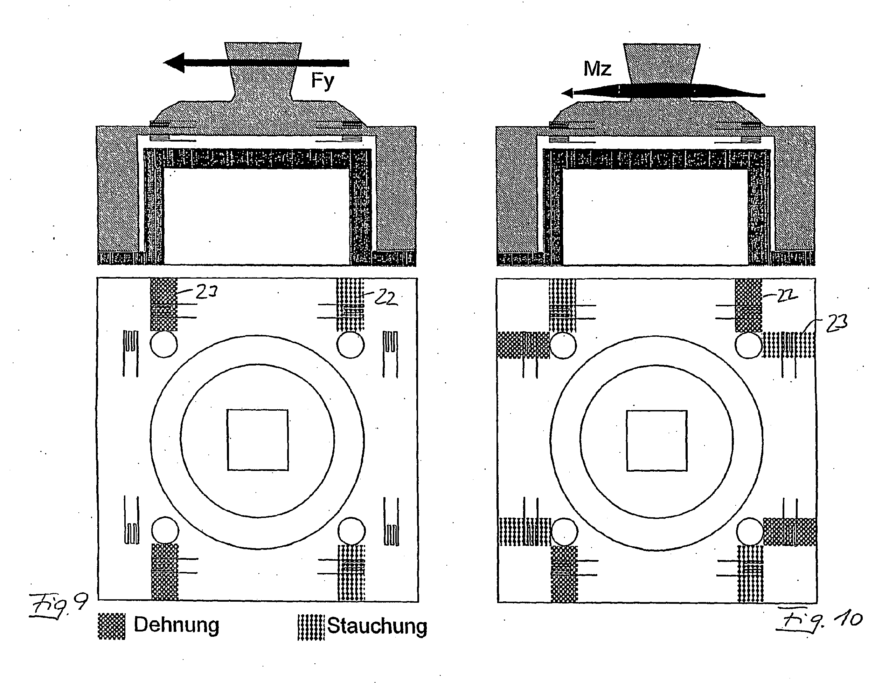

Figur 9 zeigt eine auf den Justieradapter 10 einwirkende seitliche Kraft in der Zeichenebene (y-Richtung), durch die nur senkrecht zu der angreifenden Kraft stehende Dehnungsbereiche 23 und Stauchungsbereiche 22 gebildet werden, während die übrigen Dehnungsmessstreifen 21 auf der Oberseite 17 des zweiten Flansches 12 ohne Messsignal bleiben.

Bei einem in Figur 10 dargestellten angreifenden Drehmoment Mz in z-Richtung entstehen an jeder Ausnehmung 20 ein Stauchungsbereich 23 und ein Dehnungsbereich 22, wobei in Richtung des Drehmoments Mz gesehen, der Stauchungsbereich 23 jeweils um 90° dem Dehnungsbereich 22 vorläuft.

Anhand der dargestellten Beispiele ist erkennbar, dass mit den Dehnungsmessstreifen 21 als Sensorelemente die verschiedenen auftretenden Kräfte und Momente eindeutig detektierbar sind.

Die Dehnungsmessstreifen 21 an der Unterseite des zweiten Flansches 12 ergeben jeweils Signale, die komplementär zu den Signalen der Dehnungsmessstreifen 21 an der Oberseite 17 des zweiten Flansches 12 sind, sodass sie bei einer geeigneten Addition zu einem verstärkten Messsignal beitragen können.

## Patentansprüche

1. Sensoranordnung für die Messung von Kräften und/oder Momenten, die als starrer Übertrager zur Übertragung der Kräfte und/oder Momente ausgebildet ist, einen ersten Teil (1) mit einem ersten Anschluss (2) und einen zweiten Teil (11) mit einem zweiten Anschluss (10) aufweist und mit mechanisch-elektrischen Sensorelementen (21) zur Umsetzung mechanischer Parameter in elektrische Parameter ausgestattet ist, **dadurch gekennzeichnet, dass** die Sensoranordnung ausgehend von dem ersten Anschluss (2) einen den ersten Anschluss (2) umgebenden ersten Flansch (3) aufweist, der über senkrecht zum ersten Flansch (3) stehende Stege (13) mit einem parallel zum ersten Flansch (3) ausgerichteten zweiten Flansch (12) verbunden ist, wobei der Zwischenraum (15) zwischen den Stegen (13) größer als Breitenabmessungen der Stege (13) ist, dass auf dem zweiten Flansch (12) der zweite Anschluss (10) angeordnet ist und dass die Sensorelemente zur Bestimmung von Dehnungen oder Stauchungen ausgelegt und auf wenigstens einer der beiden Oberflächen des zweiten Flansches (12) neben den Stegen (13) angeordnet sind.

2. Sensoranordnung nach Anspruch 1, **dadurch gekennzeichnet, dass** die Sensorelemente (21) zur Aufnahme von Dehnungen oder Stauchungen auf beiden Oberflächen des zweiten Flansches (12) angeordnet sind.

3. Sensoranordnung nach Anspruch 1 oder 2, **dadurch gekennzeichnet, dass** sie zweiteilig mit einer den ersten Flansch (3) aufweisenden ersten Teil (1) und einem den zweiten Flansch (12) aufweisenden zweiten Teil (11) aufgebaut ist und dass die Teile (1, 11) starr miteinander verbindbar sind.

4. Sensoranordnung nach Anspruch 3, **dadurch gekennzeichnet, dass** die Stege (13) mit einer Gewindebohrung (14) zur Aufnahme einer Befestigungsschraube versehen sind.

5. Sensoranordnung nach einem der Ansprüche 1 bis 4, **dadurch gekennzeichnet, dass** der erste Anschluss-(2) hutförmig mit einem zylindrischen Querschnitt ausgebildet ist, an den sich der erste Flansch (3) anschließt.

6. Sensoranordnung nach einem der Ansprüche 1 bis 5, **dadurch gekennzeichnet, dass** der zweite Anschluss (10) mit einer konvexen Wölbung in den zweiten Flansch (12) übergeht.

7. Sensoranordnung nach einem der Ansprüche 1 bis 6, **dadurch gekennzeichnet, dass** die Sensorelemente (21) Definungsmessstreifen sind.

8. Sensoranordnung nach Anspruch 7, **dadurch gekennzeichnet, dass** die Dehnungsmessstreifen (21) zur Aufnahme linienförmiger Dehnungen oder Stauchungen positioniert sind.

9. Sensoranordnung nach einem der Ansprüche 1 bis 8, **dadurch gekennzeichnet, dass** der zweite Flansch (12) Ausnehmungen (20) zur Positionierung von Dehnungsbereichen (23) und Stauchungsbereichen (22) aufweist.

10. Sensoranordnung nach einem der Ansprüche 1 bis 9, **dadurch gekennzeichnet, dass** der erste Flansch (3) und der zweite Flansch (12) eine viereckige Kontur aufweisen und dass vier Stege (13) vorgesehen sind.

11. Sensoranordnung nach Anspruch 10, **dadurch gekennzeichnet, dass** vier Ausnehmungen (20) in diagonaler Richtung zwischen dem zweiten Anschluss (10) und den Stegen (13) angeordnet sind.

12. Sensoranordnung nach Anspruch 11, **dadurch gekennzeichnet, dass** die Dehnungsmessstreifen (21) zur Aufnahme von parallel zu den Kanten des zweiten Flansches (12) von den Ausnehmungen (20) verlaufenden Dehnungen oder Stauchungen positioniert sind.

13. Sensoranordnung nach einem der Ansprüche 1 bis 12, **dadurch gekennzeichnet, dass** der zweite Anschluss (10) zentrisch zum zweiten Flansch (12) angeordnet ist.

14. Verwendung der Sensoranordnung nach einem der Ansprüche 1 bis 13 in einem künstlichen Glied, insbesondere in einem künstlichen Bein, anstelle eines ausbaubares Bauteils des künstlichen Glieds, an dessen Abmessungen die Sensoranordnung angepasst ist.

15. Verwendung der Sensoranordnung gemäß Anspruch 14 anstelle eines Rotationsadapters des künstlichen Beines.

## Claims

1. Sensor assembly for measuring forces and/or torques, which sensor assembly is designed as a rigid transmitter for transmitting the forces and/or torques, has a first part (1) with a first connection (2) and a second part (11) with a second connection (10), and is provided with electromechanical sensor elements (21) for converting mechanical parameters into electrical parameters, **characterized in that** the sensor assembly has a first flange (3) which surrounds the first connection (2), originates at the first connection (2), and is connected to a second flange (12) via struts (13) which are perpendicular to the first flange (3), said second flange (12) being aligned parallel to the first flange (3), the gap (15) between the struts (13) being larger than the width of the struts (13), **in that** the second connection (10) is arranged on the second flange (12), and **in that** the sensor elements are designed for determining strains or compressions and are arranged next to the struts (13) on at least one of the two surfaces of the second flange (12).

2. Sensor assembly according to Claim 1, **characterized in that** the sensor elements (21) for recording strains or compressions are arranged on both surfaces of the second flange (12).

3. Sensor assembly according to Claim 1 or 2, **characterized in that** it is constructed in two parts with a first part (1) having the first flange (3) and a second part (11) having the second flange (12), and **in that** the parts (1, 11) can be rigidly connected to one another.

4. Sensor assembly according to Claim 3, **characterized in that** the struts (13) are provided with a threaded bore (14) for holding a fixing screw.

5. Sensor assembly according to one of Claims 1 to 4, **characterized in that** the first connection (2) is formed in the shape of a hat with a cylindrical cross section to which the first flange (3) adjoins.

6. Sensor assembly according to one of Claims 1 to 5, **characterized in that** the second connection (10) merges into the second flange (12) with a convex bulge.

7. Sensor assembly according to one of Claims 1 to 6, **characterized in that** the sensor elements (21) are strain gauges.

8. Sensor assembly according to Claim 7, **characterized in that** the strain gauges (21) are positioned to record linear strains or compressions.

9. Sensor assembly according to one of Claims 1 to 8, **characterized in that** second flange (12) has recesses (20) for the positioning of strain regions (23) and compression regions (22).

10. Sensor assembly according to one of Claims 1 to 9, **characterized in that** the first flange (3) and the second flange (12) have a quadrangular contour and **in that** four struts (13) are provided.

11. Sensor assembly according to Claim 10, **characterized in that** four recesses (20) are arranged diagonally between the second connection (10) and the struts (13).

12. Sensor assembly according to Claim 11, **characterized in that** the strain gauges (21) are positioned to record strains or compressions from the recesses (20), said strains or compressions running parallel to the edges of the second flange (12).

13. Sensor assembly according to one of Claims 1 to 12, **characterized in that** the second connection (10) is arranged centrally with respect to the second flange (12).

14. Use of the sensor assembly according to one of Claims 1 to 13 in an artificial limb, in particular in an artificial leg, in place of a removable component of the artificial limb, the sensor assembly being adapted to said component's dimensions.

15. Use of the sensor assembly according to Claim 14 in place of a rotation adaptor of the artificial leg.

## Revendications

1. Dispositif de détection destiné à la mesure de forces et/ou de moments, qui est formé en tant qu'appareil de transmission rigide pour la transmission des forces et/ou moments, qui présente une première partie (1) avec un premier raccord (2) et une deuxième partie (11) avec un deuxième raccord (10) et qui est équipé d'éléments de détection (21) électro-mécaniques pour convertir des paramètres mécaniques en paramètres électriques, **caractérisé en ce que** le dispositif de détection présente, en partant du premier raccord (2), une première bride (3) entourant le premier raccord (2), et reliée à une deuxième bride (12) orientée parallèlement à la première bride (3) par des traverses (13) agencées perpendiculairement à la première bride (3), l'espace intermédiaire (15) entre les traverses (13) étant plus grand que des dimensions en largeur des traverses (13), **en ce que** le deuxième raccord (10) est agencé sur la deuxième bride (12) et **en ce que** les éléments de détection sont disposés pour déterminer des extensions ou des compressions et sont agencés sur l'une au moins des deux surfaces de la deuxième bride (12), près des traverses (13).

2. Dispositif de détection selon la revendication 1, **caractérisé en ce que** les éléments de détection (21) sont agencés pour capter des extensions ou des compressions sur les deux surfaces de la deuxième bride (12).

3. Dispositif de détection selon la revendication 1 ou 2, **caractérisé en ce qu'**il est construit en deux parties, avec une première partie (1) présentant la première bride (3) et une deuxième partie (11) présentant la deuxième bride (12), et **en ce que** les parties (1, 11) peuvent être reliées ensemble de façon rigide.

4. Dispositif de détection selon la revendication 3, **caractérisé en ce que** les traverses (13) sont munies d'un perçage fileté (14) pour recevoir une-vis de fixation.

5. Dispositif de détection selon l'une des revendications 1 à 4, **caractérisé en ce que** le premier raccord (2) est formé en forme de chapeau avec une coupe transversale cylindrique à laquelle se raccorde la première bride (3).

6. Dispositif de détection selon l'une des revendications 1 à 5, **caractérisé en ce que** le deuxième raccord (10) se raccorde à la deuxième bride (12) par un bombement convexe.

7. Dispositif de détection selon l'une des revendications 1 à 6, **caractérisé en ce que** les éléments de détection (21) sont des jauges d'extension.

8. Dispositif de détection selon la revendication 7, **caractérisé en ce que** les jauges d'extension (21) sont positionnées pour recevoir des extensions ou compressions selon une ligne.

9. Dispositif de détection selon l'une des revendications 1 à 8, **caractérisé en ce que** la deuxième bride (12) présente des évidements (20) pour le positionnement de zones d'extension (23) et zones de compression (22).

10. Dispositif de détection selon l'une des revendications 1 à 9, **caractérisé en ce que** la première bride (3) et la deuxième bride (12) présentent un contour quadrangulaire et **en ce que** quatre traverses (13) sont prévues.

11. Dispositif de détection selon la revendication 10, **caractérisé en ce que** quatre évidements (20) sont agencés en direction diagonale entre le deuxième raccord (10) et les traverses (13).

12. Dispositif de détection selon la revendication 11, **caractérisé en ce que** les jauges d'extension (21) sont positionnées pour recevoir des extensions ou compressions s'étendant depuis les évidements (20) parallèlement aux bords de la deuxième bride (12).

13. Dispositif de détection selon l'une des revendications 1 à 12, **caractérisé en ce que** le deuxième raccord (10) est agencé centralement par rapport à la deuxième bride (12).

14. Utilisation du dispositif de détection selon l'une des revendications 1 à 13 dans un membre artificiel, en particulier dans une jambe artificielle, à la place d'un élément démontable du membre artificiel, aux dimensions duquel le dispositif de détection est adapté.

15. Utilisation du dispositif de détection selon la revendication 14 à la place d'un adaptateur en rotation de la jambe artificielle.
